# EUROPEAN PATENT SPECIFICATION

(11) **EP 4 059 547 B1**
(45) Date of publication and mention of the grant of the patent: **08.10.2025**
(21) Application number: 21163587.5
(22) Date of filing: 19.03.2021
(51) Int. Cl.: A61M 5/20, A61M 5/31, A61M 5/315, A61M 5/24

(54) **CONTACT DETECTION SYSTEM FOR AN INJECTION DEVICE**
KONTAKTDETEKTIONSSYSTEM FÜR EINE INJEKTIONSVORRICHTUNG
SYSTÈME DE DÉTECTION DE CONTACT POUR UN DISPOSITIF D'INJECTION

(43) Date of publication of application: 21.09.2022
(73) Proprietor: Ypsomed AG, 3401 Burgdorf (CH)
(72) Inventor: Brügger, Martin, 3065 Bolligen (CH); Schrul, Christian, 3400 Burgdorf (CH); Scheurer, Simon, 3006 Bern (CH); Bosshard, Simon Martin, 3006 Bern (CH)
(74) Representative: Kleiner, Stefan

(56) References cited:
- US-A1- 2005 171 476
- US-A1- 2014 114 277
- US-A1- 2019 091 410

## Description

### FIELD OF THE INVENTION

The present invention relates to injection devices for injecting, delivering, administering or dispensing substances and/or liquids such as insulin, hormone preparations or vaccines. It departs from an injection device comprising a movable reservoir element, a dispensing member and a motor adapted to move the dispensing member according to a reference motor value.

### BACKGROUND OF THE INVENTION

A variety of diseases exist that require regular treatment by subcutaneous administration of a medicament, and a number of drug delivery devices have been developed to support a patient in accurately and controllably delivering an amount of drug in a self-administration process. Delivery devices include injection devices that are removed from the injection site after each medication event or drug delivery process, as well as infusion devices with a cannula or needle that remains in the skin of the patient for a prolonged period of time.

By way of example, diabetes may be treated by self-administration of insulin or its derivatives with the help of multi-variable-dose insulin injection pens. An injection pen device generally has an elongate device body defining a longitudinal main device axis. An automatic injection device has a motor or a drive spring for biasing a plunger rod and shifting a piston in a container barrel, wherein the drive spring may have to be charged or strained manually prior to injection of a dose. A manually powered delivery drive requires a user to manually provide the energy to move the piston, for instance by applying a distal force component to the injection device.

The medicament dose to be injected may typically be manually selected by turning a dosage knob and observing the actual dialed dose from a dose window or display of the insulin pen. A dose is dispensed by inserting the needle into a suited portion of human skin and by moving the piston manually or by pressing a release button of an automatic injection device. Automatic injection devices may comprise an electronic dose dial mechanism to automatically set a dose.

Disposable or single-use delivery devices are adapted to deliver a drug from a container such as a pre-filled syringe or cartridge that is not intended to be replaced or refilled by the patient. Reusable, semi-disposable, or hybrid delivery devices have a reservoir or container that may be replaced by the patient, or a reservoir that may be refilled, while some components of the device may be reused with the replaced or refilled drug reservoir.

Such devices with reusable components have to be prepared for a new injection after replacement of the reservoir. Namely, a plunger rod or any dispensing member has to be moved towards a piston inside the reservoir, usually from a retracted position to a piston contacting position. The positioning of the plunger rod or dispensing member relative to the piston of the reservoir after a new reservoir has been inserted in the device is crucial. The plunger rod or dispensing member has to be aligned or has to be in contact with the piston in order to ensure an accurate dispensing of the set dose.

EP 2 110 150 B2 discloses a reusable injection device with a monitoring circuit. During positioning of a plunger rod relative to a piston inside the cartridge the monitoring circuit monitors an amperage of the motor. The plunger rod is moved by the motor out of a retracted position and is brought into contact with the piston. The contact causes the amperage of the motor to increase which is detected by the monitoring circuit and thus the contact position can be determined. US2019/091410, US2014/114277 and US 2005/171476 disclose further examples of such injection devices.

With such an approach the motor has to be stopped immediately if a dispensing member abuts or contacts the piston as otherwise the piston would be moved relative to a reservoir housing causing an undesirably dispensing of liquid drug. However, such an immediate stopping of the motor may be challenging as a control circuit shows a reaction time or a lag time.

### DESCRIPTION OF THE INVENTION

It is an objective of the invention to provide a reliable and safe positioning of a dispensing member relative to a reservoir in an injection device.

This objective is achieved by the injection device and the method according to the independent claims. Preferred embodiments are evident from the dependent claims.

According to the invention an injection device for injecting a liquid drug from a reservoir comprises the reservoir housing with a reservoir element movable relative to a reservoir housing, a dispensing member, a motor adapted to drive or move the dispensing member towards the reservoir element and a controller. The controller is provided with or has access to a reference motor value (a predefined input value or motor control parameter) and a captured or measured instantaneous motor value (continually measured or continually received motor operation feedback value). The controller is adapted to determine or calculate a relationship between the reference motor value and the instantaneous motor value.

The moving dispensing member is brought into physical contact with the reservoir element by the motor operated by the reference motor value. Upon contact the determined relationship between the reference motor value and the captured motor value changes. In other words, the captured instantaneous motor value changes in an unexpected manner (e. g. increasing discrepancy) with respect to the reference motor value. The controller is adapted to detect the contact between the dispensing member and the reservoir element based on the change of the relationship. A force exerted by the dispensing member, when driven by the motor according to the reference motor value, onto the reservoir element is below a breakaway force or initial force required to overcome a static friction force of the reservoir element in order to move initially the reservoir element.

The movement of the dispensing member is stopped by the reservoir element and thus the dispensing member cannot be moved any more according to the reference motor value. The stopping of the dispensing member causes a change of the instantaneous motor value, for example, a change in the back-EMF, a position discrepancy or a change in the motor speed relative to the reference motor value (input value). This allows to reliably detect when the dispensing member runs into, abuts or contacts the reservoir element.

As the dispensing member can only exert a force to the reservoir element that is smaller than a breakaway force required to move the reservoir element the latter is not moved. In other words, the force required to overcome the static friction force of the reservoir element is higher than the motor force. The motor's current draw is preferably limited based on the reference motor value. Consequently, if the dispensing member abuts or contacts the reservoir element the motor cannot move the reservoir element and therefore no liquid drug is dispensed from the reservoir during the position detection process according to the invention.

The injection device comprises preferably a reservoir including a reservoir housing and reservoir element. The reservoir element is moveable relative to the reservoir housing to dispense the liquid drug from the reservoir. The reservoir element can be, for example, a piston or a plunger inside the reservoir or an actuation element of the reservoir for dispensing the drug. The dispensing member is a component of the drive mechanism, preferably of a reusable drive unit. The dispensing member may be, for example, a plunger rod, a drive sleeve, a coupling sleeve or an interface member adapted to couple the motor to the piston.

The reference motor value and the instantaneous motor value can be the same type of parameter or they may be a different type of parameter. The reference motor value is a predefined value or input value. Examples of the reference motor value are an input current, an input voltage, a speed level for the motor or a pulse width or frequency for a chopper circuit for driving the motor. In contrast, the captured instantaneous motor value is a continuously measured motor feedback value determined by one or more device sensors or a captured value received from an external sensor or device. The captured instantaneous motor value may be, for example, an instantaneously consumed motor current, a motor voltage, a rotor position, a position of a dispensing member, motor speed or a back-EMF value.

The controller is adapted to determine a relationship between the reference motor value and the captured motor value. Such a relationship may be, for example, a mathematical relation (e. g. one value divided by the other value), a mathematical function (linear, exponential) or a determined difference between the two values. The relationship is constant or changes in a predictable and expectable manner as long as the dispensing member is moving according to the reference motor value. The relationship changes or changes in an unexpected manner if the moving dispensing member is stopped by the reservoir element. Such a change may be, for example, an increasing discrepancy of the expected position to a captured (measured) position, an unexpected change of the back-EMF, an unexpected increase or decrease of the motor draw current or voltage or any other unexpected change of a motor feedback value or motor parameter.

In the present context, the terms "substance", "drug", "medicament" and "medication" are to be understood to include any flowable medical formulation suitable for controlled administration through a means such as, for example, a cannula or a hollow needle, and comprises a liquid, a solution, a gel or a fine suspension containing one or more medical active ingredients. A medicament can be a composition comprising a single active ingredient or a pre-mixed or co-formulated composition with more than one active ingredient present in a single container. Medication includes drugs such as peptides (e.g., insulin, insulin-containing drugs, GLP-1 containing drugs or derived or analogous preparations), proteins and hormones, active ingredients derived from, or harvested by, biological sources, active ingredients based on hormones or genes, nutritional formulations, enzymes and other substances in both solid (suspended) or liquid form but also polysaccharides, vaccines, DNA, RNA, oligonucleotides, antibodies or parts of antibodies but also appropriate basic, auxiliary and carrier substances

The term "distal" is meant to refer to the direction or the end of the injection device carrying an injection needle or an injection cannula, whereas the term "proximal" is meant to refer to the opposite direction or end pointing away from the needle or cannula.

The term "injection system" or "injector" refers to a device that is removed from the injection site after each medication event or drug delivery process (for example an injection pen), whereas the term "infusion system" refers to a device with a cannula or needle that remains in the skin of the patient for a prolonged period of time, for example, several hours (for example a drug pump).

Examples for an injection device may be a disposable injection pen comprising a cartridge with the liquid drug, a semi-disposable injection pen with a reusable drive unit and a replaceable cartridge unit comprising a reservoir, a reusable injection pen with replaceable reservoir, an autoinjector comprising a syringe (in particular a semi-disposable autoinjector with a reusable drive unit and a replaceable receptacle including a syringe) or a patch injector.

In a preferred embodiment the reference motor value is a motor input current defining or limiting a output force such that the output force is below a pre-determined threshold, preferably the output force is below the breakaway force required to initially move the reservoir element. The motor input current thus restricts the motor output force to a specific maximum. For a rotating motor the output is a maximum motor torque and for a linear motor the output is a maximum linear force in a dispensing direction.

Alternatively, the reference motor value can be a motor speed level, a motor voltage, a pulse width or frequency for a chopper circuit for driving the motor.

The captured instantaneous motor value is preferably a position value determined by an encoder. The rotational or linear encoder provides a position of a motor output member such as a rotor or a dispensing member of the motor. The controller is adapted to compare the encoder signal with the reference motor value (input signal) in order to detect a change in the relationship and thus a contact of the dispensing member with the reservoir element. In case of contact the position value does not change and thus indicating that the dispensing member has been stopped by the reservoir element.

In an alternative embodiment the captured instantaneous motor value is preferably a value of the back electromotive force (back-EMF). As the back-EMF is proportional to the rotational speed of an armature of the motor the motor speed and position can be indirectly measured via back-EMF. That means the controller is configured to detect a change in the relationship between the back-EMF and the reference motor value. Thus, the controller detects in particular an unexpected change of the back-EMF while the reference motor value (current, speed, frequency) does not change. The unexpected change in the back-EMF indicates that the dispensing member has been brought into contact with the reservoir element and the dispensing member has thus been stopped.

Additionally, the controller is preferably adapted to determine a position of the reservoir element relative to a reservoir housing based on the detected contact and based on predefined reservoir information. The controller may receive such reservoir information from a cartridge tag, from an external device or the information may be stored in the injection device. The reservoir information may comprise information about at least one of the volume, size and type of the reservoir. The reservoir information together with the detected stop position of the dispensing member allows the controller to calculate a position of the dispensing element relative to the reservoir housing. The determined position defines the remaining volume in the reservoir and thus the liquid drug remaining in the reservoir.

Preferably, the controller is adapted to stop the motor upon the detected contact. After abutting the reservoir element the dispensing member is not movable any further in the dispensing direction. Preferably, the controller immediately stops driving the motor. For a subsequent priming or injection the motor is driven to output a higher force to be able to move the reservoir element and thus to dispense drug from the reservoir.

The motor of the injection device is preferably a stepper motor. In this embodiment. A motor drive circuit sends a drive signal to rotate a rotor a fixed angle (rotation motor) or to move a motor element a defined distance (linear motor) according to the drive signal. The controller comprises a drive circuit, for example, a chopper drive circuit to generate a controlled current. An angular or linear displacement and a motor speed can be controlled by an output signal of the drive circuit.

As soon as the dispensing member abuts the reservoir element the moving motor element (rotor or linear dispensing member) cannot follow any more the drive signal. That means the motor element is not any more moving according to the commanded steps (step loss). Such a discrepancy between expected motor steps and actually carried out motor steps can be detected by the controller, for example, via position encoder or back-EMF.

Examples of stepper motors are a permanent magnet stepper motor, a variable reluctance stepper motor or a hybrid synchronous stepper motor. Alternatively, the motor may be a brushed DC motor or a brushless DC motor or a synchronous motor.

The reservoir element is preferably a piston inside the reservoir and movable relative to a reservoir housing and the dispensing member is preferably a plunger rod adapted to move the piston. Preferably, the plunger rod comprises a distal contact area configured to abut or contact directly or indirectly via an intermediate member (e. g. a flange) the piston of the reservoir and configured to move the piston. The plunger rod is not permanently connected to the piston. In a preferred embodiment the reservoir is a cartridge. The plunger rod is either directly moved by a moving motor element or indirectly via an intermediate member such as a clutch member or drive member.

In a preferred embodiment the injection device comprises a reservoir holder or cartridge holder and the injection device further includes a needle mounting portion adapted to be connected to an injection needle. The injection device is preferably pen-shaped.

Furthermore, the injection device preferably comprises a drive unit including the motor, the controller and the dispensing member. Preferably the injection device further comprise a reservoir unit or cartridge unit including the reservoir housing and the movable reservoir element. The cartridge unit may be implemented as a disposable component that is disposed of after use or after the reservoir is empty. In contrast, the drive unit may be implemented as a reusable component that can be used for a long time for a plurality of injections and can be connected to several cartridge units. The cartridge unit is preferably releasably attachable to the drive unit, for example, by a bayonet connection, a threaded connection or a snap-fit.

The invention further comprises a method for detecting a contact between a dispensing member and a reservoir element of an injection device, the method comprising the steps of
a) moving the dispensing member towards the reservoir element, by an injection device motor, with a predefined motor output force according to a reference motor value;
b) determining, by a controller, a relationship between the reference motor value and a captured instantaneous motor value;
c) physically contacting the reservoir element with the dispensing member, wherein a force exerted by the dispensing member to the reservoir element is smaller than a force required to move the dispensing element;
d) detecting the contact based on a change of the relationship;
e) stopping the motor upon contact detection.

In a preferred embodiment the method further comprises the steps of
f) registering, by the controller, upon contact a first position of the dispensing member;
g) moving back and subsequently moving forward the dispensing member towards the reservoir element;
h) detecting, by the controller, a second contact between the dispensing member and the reservoir element based on a second change of the relationship;
i) registering upon second contact a second position of the dispensing member;
j) calculating a mean position with at least the first position and the second position.

The detection of at least a first and a second contact (or even third or four contacts) is advantageous because the contact position can be confirmed by at least a second measurement. The dispensing member or the whole dispensing mechanism may be biased upon the first contact and hence the detected contact position of the piston relative to a reservoir housing may not be accurate. By generating more than one contacts the contact position may be determined based on a mean value which is more accurate than a single measurement.

### BRIEF DESCRIPTION OF THE DRAWINGS

The subject matter of the invention will be explained in more detail in the following text with reference to preferred exemplary embodiments which are illustrated in the attached drawings, in which:
- Fig. 1: depicts a top view of a semi-disposable injection pen according to the invention;
- Fig. 2: depicts a perspective view of the pen of figure 1, wherein the disposable assembly and the reusable assembly are detached from each other;
- Fig. 3: depicts a sectional view of the injection pen;
- Fig. 4: depicts a schematic view of the operation of the injection device motor.

The reference symbols used in the drawings, and their primary meanings, are listed in summary form in the list of designations. In principle, identical parts are provided with the same reference symbols in the figures.

### DETAILED DESCRIPTION OF PREFERRED EMBODIMENTS

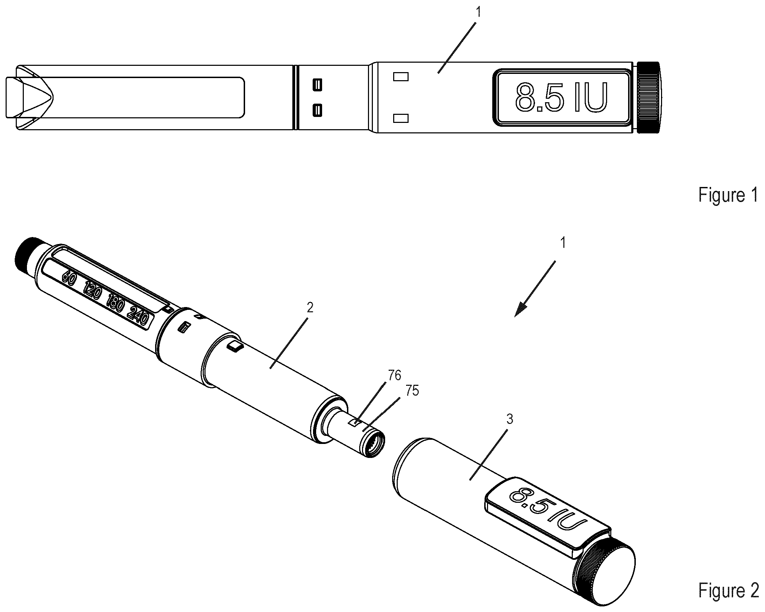

In the present description the term "distal" refers to the side of the device where a needle is attachable. This is on the left hand side in the figures 1 to 3. The term "proximal" refers to the opposite side and is on the right hand side in figures 1 to 3.

Figure 1 shows an injection device according to the invention. In the embodiment shown in figure 1 the device is implemented as a semi-disposable injection pen 1. The pen 1 comprises a disposable assembly and a reusable assembly 3.

Figure 2 depicts the injection pen of figure 1 wherein the disposable assembly is detached from the reusable assembly 3. The disposable assembly is formed by a cartridge unit 2 (or dispensing unit) including a reservoir holder 10 or cartridge holder and a reservoir in form of a cartridge 5 containing liquid drug. The reusable assembly 3 includes a drive unit and a LCD unit with a display 65. The reusable assembly 3 is releasably attachable to the cartridge unit 2.

The cartridge unit 2 can be produced, pre-assembled and stored separately from the reusable assembly 3. That is, single parts or components of the cartridge unit 2 may be produced by a device manufacturer and delivered to the drug or medication manufacturer. The latter inserts the filled cartridge 5 in the reservoir holder 10 and pre-assembles the single components to the cartridge unit 2.

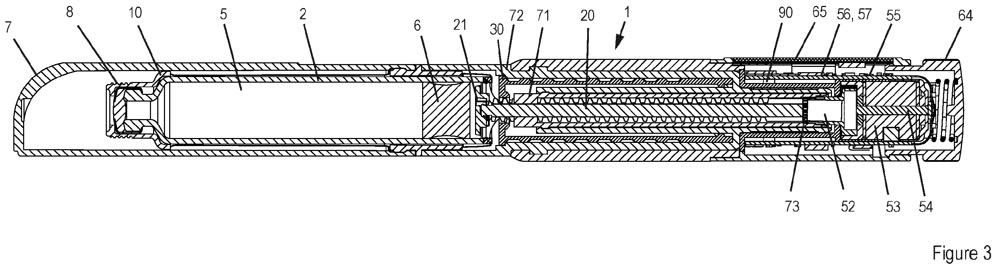

Figure 3 depicts a sectional view of the injection pen 1 wherein the cut runs along a longitudinal axis of the injection pen 1. As shown in figure 3 the cartridge unit 2 includes an interface housing 72, the reservoir holder 10, the cartridge 5, a mechanics holder 30, a plunger rod driver 71 and a plunger rod 20 with a flange 21 (dispensing member) pivotally mounted on a distal end of the plunger rod 20. The plunger rod driver 71 is coaxially arranged and rotationally guided inside the housing 72. The plunger rod 20 is further rotationally coupled but axially movable relative e to the plunger rod driver 71. At the proximal end the plunger rod driver 71 comprises circumferentially arranged teeth 73 adapted to be rotationally coupled to a drive member 52 of the automatic drive unit 50 in the reusable assembly.

The cartridge holder 10 comprises on a distal end a needle mounting portion 8 adapted to be connected to a needle assembly (not shown). A pen cap 7 can be mounted to cover the distal end of the injection pen 1.

The reusable assembly 3 comprises an automatic drive unit and the LCD unit. The automatic drive unit comprises a stepper motor 53 with a motor shaft 54 (rotor) for moving the plunger rod driver 71 and the plunger rod 20, the sleeve-shaped automatic drive member 52, a gear 59 connecting the motor shaft 54 to the automatic drive member 52, a printed circuit board (PCB) 55 with a controller 50 for controlling the motor 53, an energy source in form of a battery (not shown), a tag reader 90, a data storage module 57, a communication module 56, a rotary encoder 58 for sensing the movement of the motor shaft 54 and thus the movement of the plunger rod 20. The LCD unit includes the LCD 65 and a dose knob 64.

In order to attach the disposable cartridge unit 2 to the reusable assembly 3 the proximal cylindrical portion of the interface member 70 is inserted into the sleeve-shaped distal display unit housing. During insertion proximal teeth 73 of the plunger rod driver 71 get into contact with distal teeth of the automatic drive member 52 and thereby rotationally coupling the plunger rod driver 71 to the drive member 52. The cartridge unit 2 is further mechanically connected to the reusable assembly 2 by a bayonet connection.

When a user attaches the cartridge unit 2 to the reusable assembly 3 the injection pen 1 has to be prepared for an injection. That is, the flange 21 of the plunger rod 20 has be brought into contact with the piston 6 inside the cartridge 5. As shown in figure 3 there may be a gap between the flange 21 and the piston 6. This gap may result from manufacturing tolerances, it may arise during assembly of the cartridge unit 2 or the cartridge may not be completely filled. However, in order to be able to dispense accurately a set dose the flange 21 of the plunger rod 20 has to be in contact with the piston 6 before the injection starts.

Therefore, when attaching the cartridge unit 2 to the reusable assembly 3 the first step is to bring the distal surface of the flange 21 into contact with a proximal surface of the piston 6. For that purpose, the controller drives the motor 53 to move the plunger rod 20 in a distal or dispensing direction.

The controller may receive data regarding the position of the piston 6 from read tag information included in the tag of the cartridge unit 2. Furthermore, if the cartridge unit was already attached on the reusable assembly on an earlier time and is then reattached the controller has stored the previous plunger rod position (and thus the piston position) for the particular cartridge unit 2.

As for this preparing step the piston 6 is not supposed to be moved the controller drives the motor 23 with a low output torque. Namely, the input current of the motor 23 is controlled to a specific maximum (limiting) value such that the motor output force in the dispensing direction is smaller than a breakaway force required to move the piston 6 for a first time. That means the output force of the motor in this preparing movement is not high enough to overcome the frictional force of the piston 6.

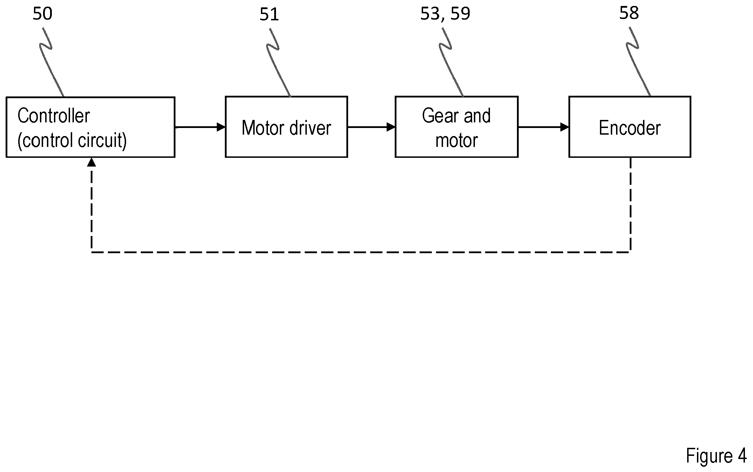

In the following the detection of the contact between the flange 21 of the piston rod 20 and the piston 6 of the cartridge 5 is described with respect to figure 4 which schematically shows a control arrangement for the stepper motor 53.

The controller 50 is provided with a motor control circuit and supplied with energy from the battery of the drive unit. The motor control circuit generates an input signal for operating the stepper motor 53 and supplies an input current to the motor driver 51.

The motor driver 51 comprises a chopper circuit for generating a variable (e.g. pulse width modulated) voltage output to be applied to the windings of the stepper motor 53. The motor input current is controlled to a specific value such that the output torque of the motor or the force of the plunger rod in the dispensing direction is smaller than the breakaway force required to move initially the piston 6 as descripted above.

Based on a reference motor signal in form of an input current the controller 50 determines an expected number of incremental rotations of the motor shaft 54 (expected motor steps). During operation of the motor the controller 50 is provided with measured incremental rotations of the motor shaft 54 (measured motor steps) from the rotational encoder 58. This feedback signal from the encoder 58 to the controller 50 is depicted as a dotted line in figure 4.

Consequently, the controller calculates or continuously monitors a relation between the expected number of motor steps and measured number of motor steps.

As soon as the distal surface of the flange 21 contacts or abuts the proximal surface of the piston 6 the flange 21 and the plunger rod 20 cannot move any further in the distal direction because the maximum force that can be exerted by the plunger rod 20 is smaller than the force required to overcome the friction of the piston 6. Therefore, the piston 5 does not move upon contact with the flange 21.

However, the controller 50 immediately detects that the commanded or expected motor steps do no longer correspond to the measured motor steps as the encoder 58 signals no movement of the motor shaft 54. Based on this detection the controller 50 stops driving the motor and registers that the flange 21 must be in contact with the piston 6. The instantaneous plunger rod position based on the encoder signal is registered as contact position. The injection pen 1 is now ready for the dispensing of a dose or for a priming process.

The drive unit may additionally include a mean contact position determination. In this case the controller 50 drives the motor 53 to retract the plunger rod 20 with the flange 21 in a proximal direction after the flange 21 has contacted the piston 6. That means the stepper motor 53 rotates a few motor steps back. Subsequently, the controller 50 drives the motor a few steps forward (distal direction) to bring the flange 21 again into contact with the piston 6. The controller 50 repeats this procedure to register further contact positions (e. g. position of the plunger rod upon contact determined based on the encoder signal). Subsequently, the controller calculates a mean contact position with the registered first, second or more contact position. This allows to determine an accurate contact position.

In an alternative embodiment the detection of the contact between the flange 21 of the plunger rod 20 and the piston 6 is based on an unexpected change of the back-EMF in the motor. For this purpose, the motor 53 comprises a sensor (not shown) for monitoring the back-EMF. The controller determines a relation between the motor input current and the back-EMF during normal operation of the motor 53, e.g. when the motor 53 drives the plunger rod 20 in distal direction. Upon contact of the flange 21 with the piston 6 the plunger rod 20 stops and thus the motor shaft 54 stops rotating. Accordingly, the back-EMF value sensed by the sensor changes in an unexpected manner and the controller 50 detects this unexpected change of the back-EMF with respect to the input current. Based on this change the contact of the flange 21 with the piston 6 can be detected. All other functions and components are similar to the first embodiment described above.

When a cartridge unit 2 is attached to the reusable assembly 3 the tag reader 90 of the drive unit reads the drug information provided in a NFC tag on the cartridge unit 2. The information comprises drug information and information about the volume and type of the cartridge 5. With this information and with the registered contact position the controller 50 is able to calculate the positon of the plunger rod 20 (and thus the positon of the piston 6) relative to an outer housing of the cartridge 5. Hence, the controller 50 is able to calculate the remaining volume and the remaining drug inside the cartridge 5. This may be in particular advantageous if a user has detached the cartridge unit 2 from the reusable assembly 3 and reattached at a later time.

While the invention has been described in detail in the drawings and foregoing description, such description is to be considered illustrative or exemplary and not restrictive. Variations to the disclosed embodiments can be understood and effected by those skilled in the art and practising the claimed invention, from a study of the drawings, the disclosure, and the appended claims. In the claims, the word "comprising" does not exclude other elements or steps, and the indefinite article "a" or "an" does not exclude a plurality. The mere fact that certain elements or steps are recited in distinct claims does not indicate that a combination of these elements or steps cannot be used to advantage, specifically, in addition to the actual claim dependency, any further meaningful claim combination shall be considered disclosed.

**LIST OF DESIGNATIONS**

| | | | |
|---|---|---|---|
| 1 | Injection pen | 50 | Controller |
| 2 | Cartridge unit (disposable assembly) | 51 | Motor driver |
| 3 | reusable assembly | 52 | Automatic drive member |
| 5 | Cartridge | 53 | Stepper motor |
| 6 | Piston | 54 | Motor shaft |
| 7 | Pen cap | 55 | PCB |
| 8 | Needle mounting portion | 56 | Communication module |
| 10 | Cartridge holder | 57 | Storage module |
| 20 | Plunger rod | 58 | Encoder |
| 21 | Flange | 59 | Gear |
| 30 | Mechanics holder | 64 | Dose knob |
| | | 65 | LCD |
| | | 71 | Plunger rod driver |
| | | 72 | Interface housing |
| | | 73 | Teeth |
| | | 90 | tag reader |

## Claims

1. An injection device (1) for injecting a liquid drug from a reservoir (5), the injection device (1) comprising
- a movable reservoir element (6);
- a dispensing member (21, 20);
- a motor (53) for moving the dispensing member (21, 20) relative to the reservoir element (6);
- a controller;
wherein the controller (50) is provided with a reference motor value for operating the motor (53) and a captured instantaneous motor value, wherein the controller (50) is adapted to determine a relationship between the reference motor value and the instantaneous motor value,
wherein when the moving dispensing member (21, 20) abuts the reservoir element (6) the relationship changes,
wherein the controller (50) is adapted to detect the contact between the dispensing member (21, 20) and the reservoir element (6) based on the change, **characterized in that** a force exerted by the dispensing member, when driven by a motor force of the motor according to the reference motor value, onto the reservoir element is below a breakaway force required to overcome a static friction force between the reservoir element (6) and the reservoir (5).

2. An injection device (1) according to claim 1, wherein the reference motor value is a motor input current defining a output force of the motor (53) below the breakaway force.

3. An injection device (1) according to claim 1 or 2, wherein the instantaneous motor value is a position value of a movable drive member determined by an encoder (58).

4. An injection device (1) according to claim 1 or 2, wherein the instantaneous motor value is a back electromotive force value.

5. An injection device (1) according to any of claims 1 to 4, wherein the controller (50) is adapted to determine a position of the reservoir element (6) relative to a reservoir housing based on the detected contact and based on predefined reservoir information.

6. An injection device (1) according to claim 5, wherein the reservoir information comprise information about at least one of the volume, size or the type of the reservoir (5).

7. An injection device (1) according to any of claims 1 to 6, wherein the controller (50) is adapted to stop the motor (53) upon the detected contact.

8. An injection device (1) according to any of claims 1 to 7, wherein the motor (53) is a stepper motor.

9. An injection device (1) according to any of claims 1 to 8, wherein the reservoir element (6) is a piston inside the reservoir (5) and the dispensing member (20, 21) is a plunger rod with a flange adapted to move the piston.

10. An injection device according to any of claims 1 to 9, wherein the device comprises the reservoir and wherein injection device includes a needle mounting portion adapted to be connected to an injection needle.

11. An injection device according to any of claims 1 to 10, wherein the device comprises
- a drive unit including the motor, the controller and the dispensing member and
- a cartridge unit including the reservoir with the reservoir element,
wherein the cartridge unit is releasably attachable to the drive unit.

12. A method for detecting a position of a reservoir element (6) relative to a dispensing member (20, 21) of an injection device (1), the method comprising the steps of
a. moving the dispensing member (20, 21), by an injection device motor (53), with a predefined motor output force according to a reference motor value;
b. determining, by a controller, a relationship between the reference motor value and a captured instantaneous motor value;
c. abutting the reservoir element (6) with the dispensing member (20, 21), **characterized in that** a force exerted by the dispensing member onto the reservoir element, when driven by a motor force of the motor according to the reference motor value, is below a breakaway force required to overcome a static friction force between the reservoir element (6) and the reservoir (5);
d. detecting the contact between the dispensing member (21, 20) and the reservoir element (6) based on a change of the relationship;
e. determining a position of the reservoir element (6) relative to a reservoir housing based on the detected contact and based on predefined reservoir information.

13. Method according to claim 12, comprising the steps of
e. registering upon contact a first position of the dispensing member (20, 21);
f. moving back and subsequently moving forward the dispensing member (20, 21) towards the reservoir element (6);
g. detecting a second contact between the dispensing member (20, 21) and the reservoir element (6) based on a second change of the relationship;
h. registering upon second contact a second position of the dispensing member (20, 21);
i. calculating a mean position of the dispensing member (20, 21) with at least the first position and the second position.

## Patentansprüche

1. Injektionsvorrichtung (1) zum Injizieren eines flüssigen Arzneimittels aus einem Reservoir (5), die Injektionsvorrichtung (1) umfassend
- ein bewegbares Reservoirelement (6);
- ein Abgabeelement (21, 20);
- einen Motor (53) zum Bewegen des Abgabeelements (21, 20) relativ zu dem Reservoirelement (6);
- eine Steuerung;
wobei der Steuerung (50) ein Referenzmotorwert zum Betreiben des Motors (53) und ein erfasster Momentanmotorwert bereitgestellt werden, wobei die Steuerung (50) angepasst ist, um ein Verhältnis zwischen dem Referenzmotorwert und dem Momentanmotorwert zu bestimmen,
wobei sich das Verhältnis ändert, wenn das bewegliche Abgabeelement (21, 20) an dem Reservoirelement (6) anliegt,
wobei die Steuerung (50) angepasst ist, um den Kontakt zwischen dem Abgabeelement (21, 20) und dem Reservoirelement (6) basierend auf der Änderung zu erkennen, **dadurch gekennzeichnet, dass**
eine Kraft, die durch das Abgabeelement, wenn es durch eine Motorkraft des Motors gemäß dem Motorreferenzwert angetrieben wird, auf das Reservoirelement ausgeübt wird, unterhalb einer Losbrechkraft liegt, die erforderlich ist, um eine statische Reibungskraft zwischen dem Reservoirelement (6) und dem Reservoir (5) zu überwinden.

2. Injektionsvorrichtung (1) nach Anspruch 1, wobei der Motorreferenzwert ein Motoreingangsstrom, der eine Ausgangskraft des Motors (53) unterhalb der Losbrechkraft definiert, ist.

3. Injektionsvorrichtung (1) nach Anspruch 1 oder 2, wobei der momentane Motorwert ein Positionswert eines bewegbaren Antriebselements, der durch einen Encoder (58) bestimmt wird, ist.

4. Injektionsvorrichtung (1) nach Anspruch 1 oder 2, wobei der Momentanmotorwert ein Wert einer gegenelektromotorischen Kraft ist.

5. Injektionsvorrichtung (1) nach einem der Ansprüche 1 bis 4, wobei die Steuerung (50) angepasst ist, um eine Position des Reservoirelements (6) relativ zu einem Reservoirgehäuse basierend auf dem erkannten Kontakt und basierend auf vordefinierten Reservoirinformationen zu bestimmen.

6. Injektionsvorrichtung (1) nach Anspruch 5, wobei die Reservoirinformationen Informationen über mindestens eines von dem Volumen, der Größe oder der Art des Reservoirs (5) umfassen.

7. Injektionsvorrichtung (1) nach einem der Ansprüche 1 bis 6, wobei die Steuerung (50) angepasst ist, um den Motor (53) bei dem erkannten Kontakt anzuhalten.

8. Injektionsvorrichtung (1) nach einem der Ansprüche 1 bis 7, wobei der Motor (53) ein Schrittmotor ist.

9. Injektionsvorrichtung (1) nach einem der Ansprüche 1 bis 8, wobei das Reservoirelement (6) ein Kolben im Inneren des Reservoirs (5) ist und das Abgabeelement (20, 21) eine Kolbenstange mit einem Flansch ist, der angepasst ist, um den Kolben zu bewegen.

10. Injektionsvorrichtung nach einem der Ansprüche 1 bis 9, wobei die Vorrichtung das Reservoir umfasst und wobei die Injektionsvorrichtung einen Nadelbefestigungsabschnitt einschließt, der angepasst ist, um mit einer Injektionsnadel verbunden zu werden.

11. Injektionsvorrichtung nach einem der Ansprüche 1 bis 10, wobei die Vorrichtung umfasst
- eine Antriebseinheit, einschließlich des Motors, der Steuerung und des Abgabeelements, und
- eine Kartuscheneinheit, einschließlich des Reservoirs mit dem Reservoirelement,
wobei die Kartuscheneinheit an der Antriebseinheit lösbar anbringbar ist.

12. Verfahren zum Erkennen einer Position eines Reservoirelements (6) relativ zu einem Abgabeelement (20, 21) einer Injektionsvorrichtung (1), das Verfahren umfassend die Schritte
a. Bewegen des Abgabeelements (20, 21), durch einen Injektionsvorrichtungsmotor (53), mit einer vordefinierten Motorausgangskraft gemäß einem Motorreferenzwert;
b. Bestimmen, durch eine Steuerung, eines Verhältnisses zwischen dem Referenzmotorwert und einem erfassten Momentanmotorwert;
c. Anlegen des Reservoirelements (6) an das Abgabeelement (20, 21),
**dadurch gekennzeichnet, dass** eine Kraft, die durch das Abgabeelement auf das Reservoirelement ausgeübt wird, wenn es durch eine Motorkraft des Motors gemäß dem Motorreferenzwert angetrieben wird, unterhalb einer Losbrechkraft liegt, die erforderlich ist, um eine statische Reibungskraft zwischen dem Reservoirelement (6) und dem Reservoir (5) zu überwinden;
d. Erkennen des Kontakts zwischen dem Abgabeelement (21, 20) und dem Reservoirelement (6) basierend auf einer Änderung des Verhältnisses;
e. Bestimmen einer Position des Reservoirelements (6) relativ zu einem Reservoirgehäuse basierend auf dem erkannten Kontakt und basierend auf vordefinierten Reservoirinformationen.

13. Verfahren nach Anspruch 12, umfassend die Schritte
e. Registrieren einer ersten Position des Abgabeelements (20, 21) bei Kontakt;
f. Zurückbewegen und anschließendes Vorwärtsbewegen des Abgabeelements (20, 21) in Richtung des Reservoirelements (6);
g. Erkennen eines zweiten Kontakts zwischen dem Abgabeelement (20, 21) und dem Reservoirelement (6) basierend auf einer zweiten Änderung des Verhältnisses;
h. Registrieren einer zweiten Position des Abgabeelements (20, 21) bei einem zweiten Kontakt;
i. Berechnen einer mittleren Position des Abgabeelements (20, 21) mit mindestens der ersten Position und der zweiten Position.

## Revendications

1. Dispositif d'injection (1) permettant l'injection d'un médicament liquide à partir d'un réservoir (5), le dispositif d'injection (1) comprenant
- un élément de réservoir mobile (6) ;
- un organe de distribution (21, 20) ;
- un moteur (53) pour déplacer l'organe de distribution (21, 20) par rapport à l'élément de réservoir (6) ;
- un dispositif de commande ;
dans lequel le dispositif de commande (50) dispose d'une valeur de référence du moteur pour le fonctionnement du moteur (53) et d'une valeur instantanée capturée du moteur, dans lequel le dispositif de commande (50) est destiné à déterminer une relation entre la valeur de référence du moteur et la valeur instantanée capturée du moteur,
dans lequel lorsque l'organe de distribution mobile (21, 20) vient en butée contre l'élément de réservoir (6), la relation change,
dans lequel le dispositif de commande (50) est destiné à détecter le contact entre l'organe de distribution (21, 20) et l'élément de réservoir (6) en fonction du changement, **caractérisé en ce qu'**
une force exercée par l'organe de distribution, lorsqu'il est entraîné par une force motrice du moteur selon la valeur de référence du moteur, sur l'élément de réservoir est inférieure à une force de rupture nécessaire pour surmonter une force de frottement statique entre l'élément de réservoir (6) et le réservoir (5).

2. Dispositif d'injection (1) selon la revendication 1, dans lequel la valeur de référence du moteur est un courant d'entrée du moteur définissant une force de sortie du moteur (53) inférieure à la force de rupture.

3. Dispositif d'injection (1) selon la revendication 1 ou 2, dans lequel la valeur instantanée du moteur est une valeur de position d'un organe d'entraînement mobile déterminée par un encodeur (58).

4. Dispositif d'injection (1) selon la revendication 1 ou 2, dans lequel la valeur instantanée du moteur est une valeur de force contre-électromotrice.

5. Dispositif d'injection (1) selon l'une quelconque des revendications 1 à 4, dans lequel le dispositif de commande (50) est destiné à déterminer une position de l'élément de réservoir (6) par rapport à un boîtier de réservoir en fonction du contact détecté et en fonction d'informations prédéfinies sur le réservoir.

6. Dispositif d'injection (1) selon la revendication 5, dans lequel les informations sur le réservoir comprennent des informations sur au moins l'un parmi le volume, la taille ou le type du réservoir (5).

7. Dispositif d'injection (1) selon l'une quelconque des revendications 1 à 6, dans lequel le dispositif de commande (50) est destiné à arrêter le moteur (53) lorsque le contact est détecté.

8. Dispositif d'injection (1) selon l'une quelconque des revendications 1 à 7, dans lequel le moteur (53) est un moteur pas à pas.

9. Dispositif d'injection (1) selon l'une quelconque des revendications 1 à 8, dans lequel l'élément de réservoir (6) est un piston à l'intérieur du réservoir (5) et l'organe de distribution (20, 21) est une tige de piston avec une bride destinée à déplacer le piston.

10. Dispositif d'injection selon l'une quelconque des revendications 1 à 9, dans lequel le dispositif comprend le réservoir et dans lequel le dispositif d'injection comporte une partie de montage d'aiguille destinée à être reliée à une aiguille d'injection.

11. Dispositif d'injection selon l'une quelconque des revendications 1 à 10, dans lequel le dispositif comprend
- une unité d'entraînement comportant le moteur, le dispositif de commande et l'organe de distribution, et
- une unité de cartouche comportant le réservoir avec l'élément de réservoir,
dans lequel l'unité de cartouche peut être fixée de manière amovible à l'unité d'entraînement.

12. Procédé de détection d'une position d'un élément de réservoir (6) par rapport à un organe de distribution (20, 21) d'un dispositif d'injection (1), le procédé comprenant les étapes consistant à
a. déplacer l'organe de distribution (20, 21), par un moteur de dispositif d'injection (53), avec une force de sortie du moteur prédéfinie selon une valeur de référence du moteur ;
b. déterminer, par un dispositif de commande, une relation entre la valeur de référence du moteur et une valeur instantanée capturée du moteur ;
c. mettre en contact l'élément de réservoir (6) avec l'organe de distribution (20, 21), **caractérisé en ce qu'**une force exercée
par l'organe de distribution sur l'élément de réservoir, lorsqu'il est entraîné par une force du moteur selon la valeur de référence du moteur, est inférieure à une force de rupture nécessaire pour surmonter une force de frottement statique entre l'élément de réservoir (6) et le réservoir (5) ;
d. détecter le contact entre l'organe de distribution (21, 20) et l'élément de réservoir (6) en fonction d'un changement de la relation ;
e. déterminer une position de l'élément de réservoir (6) par rapport à un boîtier de réservoir en fonction du contact détecté et en fonction d'informations prédéfinies sur le réservoir.

13. Procédé selon les revendications 12, comprenant les étapes consistant à
e. enregistrer lors du contact une première position de l'organe de distribution (20, 21) ;
f. faire reculer puis avancer l'organe de distribution (20, 21) vers l'élément réservoir (6) ;
g. détecter un second contact entre l'organe de distribution (20, 21) et l'élément de réservoir (6) en fonction d'un second changement de la relation ;
h. enregistrer lors du second contact une seconde position de l'organe de distribution (20, 21) ;
i. calculer une position moyenne de l'organe de distribution (20, 21) avec au moins la première position et la seconde position.
